(19) Europäisches Patentamt
European Patent Office
Office européen des brevets

(11) Veröffentlichungsnummer : **0 026 876**
**B1**

(12) # EUROPÄISCHE PATENTSCHRIFT

(45) Veröffentlichungstag der Patentschrift : **16.02.83**

(21) Anmeldenummer : **80105725.8**

(22) Anmeldetag : **24.09.80**

(51) Int. Cl.³ : **C 07 D211/58**, C 07 D211/22, C 07 D409/12, C 07 D409/14, C 07 D401/12, A 61 K 31/445

(54) **N-Phenoxyalkylpiperidin-Derivate, Verfahren zu deren Herstellung sowie diese Verbindungen enthaltende Arzneimittel.**

(30) Priorität : **28.09.79 DE 2939292**

(43) Veröffentlichungstag der Anmeldung : **15.04.81 Patentblatt 81/15**

(45) Bekanntmachung des Hinweises auf die Patenterteilung : **16.02.83 Patentblatt 83/07**

(84) Benannte Vertragsstaaten :
**AT BE CH DE FR GB IT LI LU NL SE**

(56) Entgegenhaltungen :
**DE A 2 341 376**
**DE A 2 551 235**
**DE A 2 737 630**
**GB A 989 568**
**US A 3 691 176**

(73) Patentinhaber : **BOEHRINGER MANNHEIM GMBH**
**Sandhofer Strasse 112-132 Postfach 31 01 20**
**D-6800 Mannheim 31-Waldhof (DE)**

(72) Erfinder : **Friebe, Walter-Gunar, Dr. rer. nat.**
**Heidelberger Landstrasse 111d**
**D-6100 Darmstadt (DE)**
Erfinder : **Kampe, Wolfgang, Dr. rer. nat.**
**Zedernstrasse 49**
**D-6805 Heddesheim (DE)**
Erfinder : **Thiel, Max, Dr. rer. nat.**
**S 6,35**
**D-6800 Mannheim (DE)**
Erfinder : **Schaumann, Wolfgang, Prof. Dr. med.**
**Mönchhofstrasse 58**
**D-6900 Heidelberg (DE)**
Erfinder : **Wilhelms, Otto-Henning, Dr. rer. nat.**
**Odenwaldstrasse 25/2**
**D-6941 Weinheim-Rittenweier (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 026 876 B1**

Jouve, 18, rue St-Denis, 75001 Paris, France

N-Phenoxyalkylpiperidin-Derivate, Verfahren zu deren Herstellung sowie diese Verbindung enthaltende Arzneimittel

Die Erfindung betrifft N-Phenoxyalkyl piperidinderivate der allgemeinen Formel I

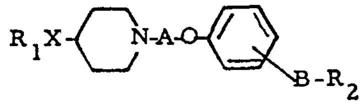

(I)

in der

$R_1$ Wasserstoff, einen $C_1$-$C_7$ Alkanoyl-Rest, einen Trifluoracetylrest, einen Phenacetyl-Rest, einen $C_3$-$C_7$ Cycloalkylcarbonyl-Rest, einen Furancarbonyl-, Thiophencarbonyl- oder Pyridincarbonyl-Rest, einen Phenyl- oder Naphthyl-Rest, oder einen Benzoyl-Rest, der durch Halogen, Hydroxyl, $C_1$-$C_6$ Alkyl, $C_1$-$C_6$ Alkoxy, $C_1$-$C_6$ Alkoxycarbonyl, Acetyloxy, Carboxyl, Nitro, Amino, Nitril, Trifluormethyl, Methylmercapto, Methylsulfinyl, Methylsulfonyl, Acetyl, Benzoyl, Carbamoyl, Acetylamino, Hydroxymethyl oder $C_1$-$C_6$ Alkoxy-$C_1$-$C_6$ alkyl substituiert sein kann,

$R_2$ eine Hydroxymethylgruppe, eine Cyanogruppe, eine Amidinogruppe, die gewünschtenfalls durch Hydroxyl substituiert sein kann, eine 1H-Tetrazol-5-yl-gruppe oder eine Gruppe —CO—$R_3$,

$R_3$ eine Hydroxygruppe, eine $C_1$-$C_6$ Alkoxygruppe oder eine Aminogruppe, die gewünschtenfalls durch 1H-Tetrazol-5-yl substituiert sein kann,

X eine Iminogruppe oder eine Oxymethylengruppe,

A einen Trimethylen-Rest und

B einen Valenzstrich oder eine 4-Hydroxy-pyrimidin-2,5-diyl-gruppe bedeuten, und deren pharmakologisch verträgliche Salze sowie Verfahren zu deren Herstellung.

Weiterhin betrifft die Erfindung pharmazeutische Präparate mit einem Gehalt an Verbindungen der allgemeinen Formel I sowie die Verwendung von Verbindungen der allgemeinen Formel I zur Herstellung solcher Präparate, wobei die erfindungsgemäßen Substanzen gegebenenfalls in der Form von Salzen nichttoxischer anorganischer oder organischer Säuren oder Basen Anwendung finden.

Die erfindungsgemäßen Substanzen wirken antiallergisch, vor allem aufgrund ihres starken Antihistamineffekts, der an Haut- und Bronchialsystem nachweisbar ist. Desweiteren sind eine antiödematöse und antiphlogistische Wirksamkeit deutlich ausgeprägt.

In der DE-OS 27 37 630 sind bereits heterocyclische Oxyalkylpiperidine mit einer Phenoxymethylgruppe in 4-Stellung beschrieben, jedoch besitzen diese Verbindungen β-blockierende Eigenschaften.

Stellt $R_1$ den Säurerest einer $C_3$-$C_7$ Cycloalkylcarbonsäure dar, so wird unter Cycloalkyl vorzugsweise der Cyclopropyl-, Cyclobutyl-, Cyclopentyl-, Cyclohexyl- oder Cycloheptyl-Rest verstanden.

Bedeutet $R_1$ Phenyl oder Naphthyl, so sind gewünschtenfalls vorhandene Substituenten Hydroxyl, Halogen, Niederalkyl oder Niederalkoxy mit 1-6 C-Atomen.

Als Halogenatome kommen Fluor, Chlor und Brom in Frage.

Außer den in den Beispielen genannten Verbindungen sind Gegenstand der Erfindung alle Verbindungen, die jede mögliche Kombination der in den Beispielen genannten Substituenten aufweisen.

Das erfindungsgemaesse Verfahren zur Herstellung der Verbindungen der allgemeinen Formel I ist dadurch gekennzeichnet, dass man in an sich bekannter Weise eine Verbindung der allgemeinen Formel II

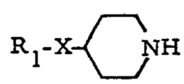

(II)

in der

$R_1$ und X die obengenannte Bedeutung haben, mit einer Verbindung der allgemeinen Formel III

$$Y_1—A—Y_2 \qquad (III)$$

in der

$Y_1$ und $Y_2$ reaktive Reste bedeuten und A die obengenannte Bedeutung hat, und einer Verbindung der allgemeinen Formel IV

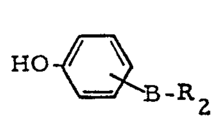

(IV)

in der

B und $R_2$ die obengenannte Bedeutung haben, umsetzt, anschliessend gewuenschtenfalls die Gruppe $R_1$ oder die Gruppe —B—$R_2$ durch an sich bekannte Verfahren in eine andere, der obengenannten Bedeutung entsprechende Gruppe $R_1$ oder —B—$R_2$ umwandelt und das so erhaltene Reaktionsprodukt gewuenschtenfalls in ein pharmakologisch vertraegliches Salz ueberfuehrt.

Als reaktive Reste $Y_1$ und $Y_2$ der Verbindungen der allgemeinen Formel III kommen Chlor, Brom, Mesyloxy und Tosyloxy in Frage.

Das erfindungsgemaesse Verfahren fuehrt man beispielsweise so durch, dass man zunaechst Verbindungen der allgemeinen Formel III mit Verbindungen der allgemeinen Formel IV kondensiert und das so erhaltene Reaktionsprodukt isoliert. Dieses Zwischenprodukt wird dann mit einer Verbindung der allgemeinen Formel II zur Reaktion gebracht. Die Umsetzung erfolgt zweckmaessig in alkalischem Medium, vorzugsweise einem niederen Alkohol wie z. B. Isopropanol in Anwesenheit von Natriumisopropanolat.

Eine andere Variante besteht darin, zunaechst Verbindungen der allgemeinen Formel II mit Verbindungen der allgemeinen Formel III zur Reaktion zu bringen ; anschliessend wird das so erhaltene Reaktionsgemisch mit Verbindungen der allgemeinen Formel IV zum gewuenschten Endprodukt der allgemeinen Formel I umgesetzt.

Eine nachtraegliche Umwandlung der Gruppe $R_1$ in der allgemeinen Formel I in eine andere Gruppe $R_1$ erfolgt beispielsweise durch Acylierung einer Verbindung der Formel I, in der $R_1$ fuer Wasserstoff steht, mit einer Verbindung $R_1$—Z, wobei Z einen reaktiven Rest darstellt. Reaktive Reste Z koennen alle Reste sein, die in der Peptidchemie zur Aktivierung von Carbonsaeuren Verwendung finden, beispielsweise Halogenatome, die Azido-Gruppe, Alkyloxy-, Aryloxy- und Acyloxy-Gruppen.

Eine nachtraegliche Umwandlung der Gruppe B—$R_2$ erfolgt beispielsweise durch saure oder alkalische Hydrolyse einer Cyanogruppe zu einer Carboxylgruppe oder Carboxamidogruppe, durch Umsetzung einer Cyanogruppe mit Stickstoffwasserstoffsaeure oder einem Salz hiervon zu einer 1H-Tetrazol-5-yl-gruppe, durch Umwandlung einer Cyanogruppe in eine Amidoximgruppe, durch Umwandlung einer Amidoximgruppe in eine Amidinogruppe, durch Veresterung einer Carboxylgruppe, durch Hydrolyse einer Estergruppe, durch Reduktion einer Estergruppe zu einer Hydroxymethylgruppe, durch Amidierung einer Carboxylgruppe zu einer Carboxamidogruppe oder einer Tetrazolylcarboxamidogruppe oder durch Reaktion einer Amidinogruppe mit einem Alkoxymethylenmalonesterderivat zu einer 5-Alkoxycarbonyl-4-hydroxy-pyrimidin-2-yl-gruppe.

Die Verbindungen der allgemeinen Formeln II, III und IV sind aus der Literatur bekannt oder können von bekannten Verbindungen ausgehend nach trivialen Methoden leicht hergestellt werden. So sind z. B. Aryloxymethyl-4-piperidine aus der DE-OS 25 49 999 bekannt.

Je nach den Verfahrensbedingungen und Ausgangsstoffen erhaelt man gegebenenfalls salzbildende Endstoffe der allgemeinen Formel I in freier Form oder in Form ihrer Salze, die sich in ueblicher Weise ineinander oder in andere Salze umwandeln lassen. So erhaelt man saure Endstoffe, wie Carbonsaeuren und Tetrazole in freier Form oder in Form ihrer Salze mit Basen. Erhaltene freie saure Verbindungen koennen in ueblicher Weise, z. B. durch Umsetzen mit entsprechenden basischen Mitteln, in die Salze mit Basen z. B. Salze mit organischen Aminen, oder Metallsalze uebergefuehrt werden. Als Metallsalze kommen vor allem Alkalimetallsalze oder Erdalkalimetallsalze, wie Natrium-, Kalium-, Magnesium- oder Calciumsalze in Betracht. Aus den Salzen lassen sich die freien Saeuren in ueblicher Weise, z. B. durch Umsetzen mit sauren Mitteln, freisetzen. Ebenso erhält man basische Verbindungen in freier Form oder in Form ihrer Salze mit Säuren. Erhaltene Salze mit Säuren können in an sich bekannter Weise, z. B. mit Alkalien oder Ionenaustauschern in die freien Verbindungen übergeführt werden. Von den letzteren lassen sich durch Umsetzung mit organischen oder anorganischen Saeuren, insbesondere solchen, die zur Bildung von therapeutisch verwendbaren Salzen geeignet sind, Salze gewinnen. Als solche Saeuren seien beispielsweise genannt : Halogenwasserstoffsaeuren, Schwefelsaeuren, Phosphorsaeuren, Salpetersaeure, Perchlorsaeure, aliphatische, alicyclische, aromatische oder heterocyclische Carbon- oder Sulfonsaeuren, wie Ameisen-, Essig-, Propion-, Bernstein-, Glykol-, Milch-, Aepfel-, Wein-, Citronen-, Ascorbin-, Malein-, Hydroxymalein- oder Brenztraubensaeure ; Phenylessig-, Benzoe-, p-Aminobenzoe-, Anthranil-, p-Hydroxybenzoe-, Salicyl- oder p-Aminosalicyl-, Embon-, Methansulfon-, Ethansulfon-, Hydroxyethansulfon-, Ethylensulfonsaeure ; Halogenbenzolsulfon-, Toluolsulfon-, Naphthalinsulfon- oder Sulfanilsaeure ; Methionin, Tryptophan, Lysin oder Arginin.

Diese und andere Salze koennen auch zur Reinigung der neuen Verbindungen verwendet werden, z. B. indem man die freien Verbindungen in ihre Salze ueberfuehrt, diese isoliert und wieder in die freien Verbindungen ueberfuehrt.

Zur Herstellung von Arzneimitteln werden die Verbindungen der allgemeinen Formel I in an sich bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z. B. Olivenoel, suspendiert oder geloest.

Die erfindungsgemäßen Verbindungen der allgemeinen Formel I und ihre Salze können in flüssiger oder fester Form enteral und parenteral appliziert werden. Hierbei kommen alle üblichen Applikationsformen in Frage, beispielsweise Tabletten, Kapseln, Dragees, Sirupe, Lösungen, Suspensionen etc. Als Injektionsmedium kommt vorzugsweise Wasser zu Anwendung, welches die bei Injektionsloesungen ueblichen Zusaetze wie Stabilisierungsmittel, Loesungsvermittler und Puffer enthaelt. Derartige Zusaetze sind z. B. Tartrat- und Citrat-Puffer, Ethanol, Komplexbildner (wie Ethylendiamintetraessigsaeure und deren nichttoxische Salze), hochmolekulare Polymere (wie fluessiges Polyethylenoxid) zur Viskositaetsregelung. Fluessige Traegerstoffe fuer Injektionsloesungen muessen steril sein und werden vorzugsweise

in Ampullen abgefuellt. Feste Traegerstoffe sind z. B. Staerke, Lactose, Mannit, Methylcellulose, Talkum, hochdisperse Kieselsaeuren, hoehermolekulare Fettsaeuren (wie Stearinsaeure), Gelatine, Agar-Agar, Calciumphosphat, Magnesiumstearat, tierische und pflanzliche Fette, feste hochmolekulare Polymere (wie Polyethylenglykole) ; fuer orale Applikation geeignete Zubereitungen koennen gewuenschtenfalls Geschmacks- und Sueßstoffe enthalten.

Die verabreichte Dosierung haengt vom Alter, der Gesundheit und dem Gewicht des Empfaengers, dem Ausmass der Krankheit, der Art gleichzeitiger gegebenenfalls durchgefuehrter weiterer Behandlungen, der Haeufigkeit der Behandlungen und der Art der gewuenschten Wirkung ab. Ueblicherweise betraegt die taegliche Dosis der aktiven Verbindung 0.1 bis 50 mg/kg Koerpergewicht. Normalerweise sind 0.5 bis 40 und vorzugsweise 1.0 bis 20 mg/kg/Tag in einer oder mehreren Anwendungen pro Tag wirksam, um die gewuenschten Resultate zu erhalten.

Neben den in den folgenden Beispielen aufgefuehrten Verbindungen werden weiterhin genannt :

5-⟨2-[3-(4-Trifluoracetamido-piperidino)-propoxy]-phenyl⟩-1H-tetrazol
5-{2-⟨3-[4-(4-Methoxycarbonyl-benzamido)-piperidino]-propoxy⟩-phenyl}-1H-tetrazol
5-{2-⟨3-[4-(4-Carboxy-benzamido)-piperidino]-propoxy⟩-phenyl}-1H-tetrazol
5-{2-⟨3-[4-(4-Cyano-benzamido)-piperidino]propoxy⟩-phenyl}-1H-tetrazol
5-{2-⟨3-[4-(3-Trifluormethyl-benzamido)-piperidino]-propoxy⟩-phenyl}-1H-tetrazol
5-{2-⟨3-[4-(4-Amincarbonyl-benzamido)-piperidino]-propoxy⟩-phenyl}-1H-tetrazol
5-{2-⟨3-[4-(4-Methylmercapto-benzamido)-piperidino]-propoxy⟩-phenyl}-1H-tetrazol
5-{2-⟨3-[4-(4-Methylsulfinyl-benzamido)-piperidino]-propoxy⟩-phenyl}-1H-tetrazol
5-{2-⟨3-[4-(4-Methylsulfonyl-benzamido)-piperidino]-propoxy⟩-phenyl}-1H-tetrazol
5-{2-⟨3-[4-(4-Acetyl-benzamido)-piperidino]-propoxy⟩-phenyl}-1H-tetrazol
5-{2-⟨3-[4-(2-Benzoyl-benzamido)-piperidino]-propoxy⟩-phenyl}-1H-tetrazol
5-{2-⟨3-[4-(2-Acetamido-benzamido)-piperidino]-propoxy⟩-phenyl}-1H-tetrazol
5-{2-⟨3-[4-(4-Hydroxymethyl-benzamido)-piperidino]-propoxy⟩-phenyl}-1H-tetrazol
5-{2-⟨3-[4-(2-Methoxymethyl-benzamido)-piperidino]-propoxy⟩-phenyl}-1H-tetrazol

Die nachfolgenden Beispiele zeigen einige der zahlreichen Verfahrensvarianten, die zur Synthese der erfindungsgemäßen Verbindungen verwendet werden können.

Beispiel 1

2-[3-(4-Phenylacetamido-piperidino)-propoxy]-benzonitril

Zu der Loesung von 1.03 g (0.045 mol) Natrium in 100 ml 2-Propanol gibt man 5.35 g (0.045 mol) 2-Hydroxy-benzonitril, erwaermt 10 Minuten zum Rueckfluss, fuegt 13.2 g (0.045 mol) 3-(4-Phenylacetamido-piperidino)-propylchlorid zu, erhitzt 6 Stunden zum Rueckfluss, engt ein, nimmt in Methylenchlorid auf, waescht mit verd. Natronlauge, engt die organische Phase ein und verreibt den Rueckstand mit Ether.

Man isoliert 12.3 g 2-[3-(4-Phenylacetamido-piperidino)-propoxy]-benzonitril (73 % d. Th.) vom Schump. 105-107 °C.

Beispiel 2

In analoger Weise wie in Beispiel 1 beschrieben erhaelt man :

| Bezeichnung | Ausbeute % | Schmelzpunkt °C (Loesungsmittel) |
|---|---|---|
| a) 2-[3-(4-Benzamido-piperidino)-propoxy]-benzoesaeuremethylester aus Salicylsaeuremethylester und 3-(4-Benzamidopiperidino)-propylchlorid | 91 | 90-92 (Cyclohexan) |
| b) 2-[3-(4-Benzamido-piperidino)-propoxy]-benzamid aus Salicylamid und 3-(4-Benzamido-piperidino)-propylchlorid | 84 | 195-196 (Ethanol) |
| c) 2-[3-(4-Benzamido-piperidino)-propoxy]-benzylalkohol aus Saligenin und 3-(4-Benzamido-piperidino)-propylchlorid | 61 | 130-131 (Essigester) |
| d) 2-[3-(4-Cyclopropancarboxamido-piperidino)-propoxy]-benzonitril aus 2-Hydroxy-benzonitril und 3-(4-Cyclopropan-carboxamido-piperidino)-propylchlorid | 73 | 123-125 (Ether) |

Beispiel 2 (Fortsetzung)

| Bezeichnung | Ausbeute % | Schmelzpunkt °C (Loesungsmittel) |
|---|---|---|
| e) 2-[3-(4-Benzamido-piperidino)-propoxy]-benzonitril aus 2-Hydroxy-benzonitril und 3-(4-Benzamido-piperidino)-propylchlorid | 67 | 143-145 (Essigester) |
| f) 2-⟨3-[4-(4-Fluor-benzamido)-piperidino]-propoxy⟩-benzonitril aus 2-Hydroxy-benzonitril und 3-[4-(4-Fluor-benzamido)-piperidino]-propylchlorid | 72 | 129-130 (Dichlormethan) |
| g) 2-⟨3-[4-(2-Methyl-benzamido)-piperidino]-propoxy⟩-benzonitril aus 2-Hydroxy-benzonitril und 3-[4-(2-Methyl-benzamido)-piperidino]-propylchlorid | 54 | 118-120 (Ether) |
| h) 3-[3-(4-Benzamido-piperidino)-propoxy]-benzonitril aus 3-Hydroxy-benzonitril und 3-(4-Benzamido-piperidino)-propylchlorid | 91 | 133-136 (Dichlormethan) |
| i) 4-[3-(4-Benzamido-piperidino)-propoxy]-benzonitril aus 4-Hydroxy-benzonitril und 3-(4-Benzamido-piperidino)-propylchlorid | 85 | 175-177 (Dichlormethan) |

## Beispiel 3

2-[3-(4-Cyclohexancarboxamido-piperidino)-propoxy]-benzonitril

Eine Mischung aus 7.35 g (0.035 mol) 4-Cyclohexancarboxamidopiperidin, 8.4 g (0.035 mol) 2-(3-Brom-propoxy)-benzonitril, 13.9 ml (0.1 mol) Triethylamin und 125 ml Tetrahydrofuran wird 6 stunden zum Rueckfluss erhitzt, anschliessend in Wasser gegossen, mit Methylenchlorid extrahiert und der Extrakt eingeengt. Es verbleiben 12.4 g 2-[3-(4-Cyclohexancarboxamido-piperidino)-propoxy]-benzonitril (97 % d. Th.) vom Schmp. 110-112 °C.

## Beispiel 4

In analoger Weise wie in Beispiel 3 beschrieben erhaelt man :

| Bezeichnung | Ausbeute % | Schmelzpunkt °C (Loesungsmittel) |
|---|---|---|
| a) 2-[3-(4-Hydroxymethyl-piperidino)-propoxy]-benzonitril aus 2-(3-Brom-propoxy)-benzonitril und 4-Hydroxy-methyl-piperidin | 77 | Oel |
| b) 2-[3-(4-Amino-piperidino)-propoxy]-benzonitril aus 2-(3-Brom-propoxy)-benzonitril und 4-Amino-piperidin | 93 | Oel |
| c) 2-[3-(4-Acetamido-piperidino)-propoxy]-benzonitril aus 2-(3-Brom-propoxy)-benzonitril und 4-Acetamido-piperidin | 58 | 103-106 (Ether) |
| d) 2-[3-(4-Isobutyramido-piperidino)-propoxy]-benzonitril aus 2-(3-Brom-propoxy)-benzonitril und 4-Isobutyramido-piperidin | 93 | 103-104 (Dichlormethan) |
| e) 2-⟨3-[4-(2-Methoxy-benzamido)-piperidino]-propoxy⟩-benzonitril aus 2-(3-Brom-propoxy)-benzonitril und 4-(2-Methoxybenzamido)-piperidin | 88 | Oel |
| f) 2-⟨3-[4-(2-Amino-benzamido)-piperidino]-propoxy⟩-benzonitril aus 2-(3-Brom-propoxy)-benzonitril und 4-(2-Amino-benzamido)-piperidin | 99 | Oel |

Beispiel 4 (Fortsetzung)

| Bezeichnung | Ausbeute % | Schmelzpunkt °C (Loesungsmittel) |
|---|---|---|
| g) 2-[3-(4-Phenoxymethyl-piperidino)-propoxy]-benzonitril aus 2-(3-Brom-propoxy)-benzonitril und 4-Phenoxy-methyl-piperidin | 90 | Oel |

Beispiel 5

2-⟨3-[4-(2-Acetoxy-benzamido)-piperidino]-propoxy⟩-benzonitril

Zu der Loesung von 11.6 g (0.045 mol) 2-[3-(4-Amino-piperidino)-propoxy]-benzonitril (Beispiel 4 b) in 100 ml Methylenchlorid fuegt man 8.4 g Natriumhydrogencarbonat und tropft die Loesung von 9.9 g (0.05 mol) 2-Acetyl-salicylsaeurechlorid zu. Man erwaermt 4 Stunden zum Rueckfluss, giesst in Wasser, extrahiert mit Methylenchlorid und engt den Extrakt ein. Nach Verreiben mit Ether verbleiben 17.0 g 2-⟨3-[4-(2-Acetoxy-benzamido)-piperidino]-propoxy⟩-benzonitril (90 % d. Th.) vom Schmp. 80-82 °C.

Beispiel 6

In analoger Weise wie in Beispiel 5 beschrieben erhaelt man :

| Bezeichnung | Ausbeute % | Schmelzpunkt °C (Loesungsmittel) |
|---|---|---|
| a) 2-[3-(4-Cyclopropancarboxamido-piperidino)-propoxy]-benzonitril (vgl. Beispiel 2 d) aus 2-[3-(4-Amino-piperidino)-propoxy]-benzonitril und Cyclopropancarbonyl-chlorid | 41 | 123-125 (Ether) |
| b 2-⟨3-[4-(2-Nitro-benzamido)-piperidino]-propoxy⟩-benzonitril aus 2-[3-(4-Amino-piperidino)-propoxy]-benzonitril und 2-Nitro-benzoylchlorid | 94 | 117-120 (Dichlormethan) |
| c) 2-[3-(4-Benzoyloxymethyl-piperidino)-propoxy]-benzonitril aus 2-[3-(4-Hydroxymethyl-piperidino)-propoxy]-benzonitril (Beispiel 4 a) und Benzoylchlorid | 98 | Oel |
| d) 2-⟨3-[4-(Thiophen-2-carboxamido)-piperidino]-propoxy⟩-benzonitril aus 2-[3-(4-Amino-piperidino)-propoxy]-benzonitril und Thiophen-2-carbonylchlorid | 66 | 97-99 (Ether) |

Beispiel 7

2-[3-(4-Benzamido-piperidino)-propoxy]-benzoesaeure

Eine Mischung aus 23.6 g (0.06 mol) 2-[3-(4-Benzamido-piperidino)- propoxy]-benzoesaeuremethylester (Beispiel 2a), 200 ml Ethanol und 200 ml N Natronlauge wird 1 Stunde zum Rueckfluss erhitzt, eingeengt, in Wasser geloest und mit verd. Salzsaeure angesaeuert. Man extrahiert mit einer Mischung aus 9 Teilen Methylenchlorid und 1 Teil Ethanol, engt den Extrakt ein, nimmt den Rueckstand in Aceton auf und faellt mit etherischer Salzaeure das Hydrochlorid. Es werden 14.5 g 2-[3-(4-Benzamido-piperidino)-propoxy]-benzoesaeure-Hydrochlorid (59 % d. Th.) vom Schmp. 207-208 °C erhalten.

Beispiel 8

2-[3-(4-Benzamido-piperidino)-propoxy]-benzoesaeure-(1H-tetrazol-5-yl)-amid

Zu einer Loesung von 5.7 g (0.015 mol) 2-[3-(4-Benzamido-piperidino)-propoxy]-benzoesaeure (Beispiel 7) in 30 ml N,N-Dimethylformamid gibt man 2.7 g (0.017 mol) N-Carbonyl-diimidazol, ruehrt 1 Stunde bei 100 °C, fuegt 1.7 g (0.02 mol) wasserfreies 5-Amino-tetrazol zu, ruehrt 3 Stunden bei 100 °C, giesst in Wasser und filtriert. Man erhaelt 3.3 g 2-[3-(4-Benzamido-piperidino)-propoxy]-benzoesaeure-(1H-tetrazol-5-yl)-amid (49 % d. Th.) vom Schmp. 259-260 °C (aus Ethanol).

Beispiel 9

5-⟨2-[3-(4-Benzamido-piperidino)-propoxy]-phenyl⟩1H-tetrazol

Eine Mischung aus 18.1 g (0.05 mol) 2-[3-(4-Benzamido-piperidino)-propoxy]-benzonitril (Beispiel 2e), 7.0 g Essigsaeure, 4.5 g (0.07 mol) Natriumazid und 75 ml n-Butanol wird 70 Stunden zum Rueckfluss erhitzt, mit weiteren 1.0 g Natriumazid und 2.0 g Essigsaeure versetzt, weitere 48 Stunden zum Rueckfluss erhitzt, darauf weitgehend eingeengt und filtriert. Man isoliert 15.2 g 5-⟨2-[3-(4-Benzamido-piperidino)-propoxy]-phenyl⟩-1H-tetrazol-Acetat vom Schmp. 260-262 °C, woraus durch Umsetzung mit verd. Natronlauge und Ansaeuren 10.6 g 5-⟨2-[3-(4-Benzamido-piperidino)-propoxy]-phenyl⟩-1H-tetrazol (53 % d. Th.) vom Schmp. 275-277 °C (aus Ethanol) erhalten werden.

Aus der vorstehenden Verbindung kann durch Neutralisation mit verd. Natronlauge und anschliessende Gefriertrocknung das Natriumsalz erhalten werden ; Ausbeute quantitativ, Schmp. 266-270 °C (amorph).

Beispiel 10

In analoger Weise wie in Beispiel 9 beschrieben erhaelt man :

| | Bezeichnung | Ausbeute % | Schmelzpunkt °C (Loesungsmittel) |
|---|---|---|---|
| a) | 5-⟨2-[3-(4-Acetamido-piperidino)-propoxy]-phenyl⟩-1H-tetrazol aus 2-[3-(4-Acetamido-piperidino)-propoxy]-benzonitril (Beispiel 4 c) und Natriumazid | 47 | 241-242 (Dimethylformamid/Ether) |
| b) | 5-⟨2-[3-(4-Isobutyramido-piperidino)-propoxy]-phenyl⟩-1H-tetrazol aus 2-[3-(4-Isobutyramido-piperidino)-propoxy]-benzonitril (Beispiel 4 d) und Natriumazid | 49 | 232-234 (Dimethylformamid/Ether) |
| c) | 5-⟨2-[3-(4-Cyclopropancarboxamido-piperidino)-propoxy]-phenyl⟩-1H-tetrazol aus 2-[3-(4-Cyclopropancarboxamido-piperidino)-propoxy]-benzonitril (Beispiel 2 d) und Natriumazid | 45 | 244-246 (Ethanol) |
| d) | 5-⟨2-[3-(4-Phenylacetamido-piperidino)-propoxy]-phenyl⟩-1H-tetrazol aus 2-[3-(4-Phenylacetamido-piperidino)-propoxy]-benzonitril (Beispiel 1) und Natriumazid | 38 | 225-226 (Isopropanol) |
| e) | 5-⟨2-[3-(4-Cyclohexancarboxamido-piperidino)-propoxy]-phenyl⟩-1H-tetrazol aus 2-[3-(4-Cyclohexancarboxamido-piperidino)-propoxy]-benzonitril (Beispiel 3) und Natriumazid | 35 | 275-277 (Dimethylformamid) |
| f) | 5-{2-⟨3-[4-(4-Fluor-benzamido)-piperidino]-propoxy⟩-phenyl}-1H-tetrazol aus 2-⟨3-[4-(4-Fluor-benzamido)-piperidino]-propoxy⟩-benzonitril (Beispiel 2 f) und Natriumazid | 44 | 276-277 (Isopropanol) |
| g) | 5-{2-⟨3-[4-(2-Methyl-benzamido)-piperidino]-propoxy⟩-phenyl}-1H-tetrazol aus 2-⟨3-[4-(2-Methyl-benzamido)-piperidino]-propoxy⟩-benzonitril (Beispiel 2 g) und Natriumazid | 31 | 250-252 (Essigester) |
| h) | 5-{2-⟨3-[4-(2-Methoxy-benzamido)-piperidino]-propoxy⟩-phenyl}-1H-tetrazol aus 2-⟨3-[4-(2-Methoxy-benzamido)-piperidino]-propoxy⟩-benzonitril (Beispiel 4 e) und Natriumazid | 39 | 194-196 (Essigester) |
| i) | 5-{2-⟨3-[4-(2-Hydroxy-benzamido)-piperidino]-propoxy⟩-phenyl}-1H-tetrazol aus 2-⟨3-[4-(2-Acetoxy-benzamido)-piperidino]-propoxy⟩-benzonitril (Beispiel 5) und Natriumazid | 42 | 268-270 (Dimethylformamid/Ether) |

Beispiel 10 (Fortsetzung)

| Bezeichnung | Ausbeute % | Schmelzpunkt °C (Loesungsmittel) |
|---|---|---|
| j) 5-{2-⟨3-[4-(2-Amino-benzamido)-piperidino]-propoxy⟩-phenyl}-1H-tetrazol aus 2-⟨3-[4-(2-Amino-benzamido)-piperidino]-propoxy⟩-benzonitril (Beispiel 4 f) und Natriumazid | 27 | 285-287 (Dimethylformamid/Ether) |
| k) 5-{2-⟨3-[4-(2-Nitro-benzamido)-piperidino]-propoxy⟩-phenyl}-1H-tetrazol aus 2-⟨3-[4-(2-Nitro-benzamido)-piperidino]-propoxy -benzonitril (Beispiel 6 b) und Natriumazid | 38 | 260-261 (Dimethylformamid/Ether) |
| l) 5-{2-⟨3-[4-(Thiophen-2-carboxamido)-piperidino]-propoxy⟩-phenyl}-1H-tetrazol aus 2-⟨3-[4-(Thiophen-2-carboxamido)-piperidino]-propoxy⟩-benzonitril (Beispiel 6 d) und Natriumazid | 48 | 264-266 (Dimethylformamid) |
| m) 5-⟨2-[3-(4-Phenoxymethyl-piperidino)-propoxy]-phenyl⟩-1H-tetrazol aus 2-[3-(4-Phenoxymethyl-piperidino)-propoxy]-benzonitril (Beispiel 4 g) und Natriumazid | 29 | 120-122 (Ethanol) |
| n) 5-⟨2-[3-(4-Benzoyloxymethyl-piperidino)-propoxy]-phenyl⟩-1H-tetrazol aus 2-[3-(4-Benzoyloxymethyl-piperidino)-propoxy]-benzonitril (Beispiel 6 c) und Natriumazid | | |
| o) 5-⟨3-[3-(4-Benzamido-piperidino)-propoxy]-phenyl⟩-1H-tetrazol aus 3-[3-(4-Benzamido-piperidino)-propoxy]-benzonitril (Beispiel 2 h) und Natriumazid | 54 | 260-262 (Dimethylformamid) |
| p) 5-⟨4-[3-(4-Benzamido-piperidino)-propoxy]-phenyl⟩-1H-tetrazol aus 4-[3-(4-Benzamido-piperidino)-propoxy]-benzonitril (Beispiel 2 i) und Natriumazid | 47 | 248-250 (Dimethylformamid/Ether) |

## Beispiel 11

2-[3-(4-Benzamido-piperidino)-propoxy]-benzamidoxim-Hydrochlorid

Eine Mischung aus 25.0 g (0.069 mol) 2-[3-(4-Benzamido-piperidino)-propoxy]-benzonitril (Beispiel 2e), 15.9 g (0.15 mol) Natriumcarbonat, 20.7 g (0.30 mol) Hydroxylamin-Hydrochlorid, 125 ml Ethanol und 175 ml Wasser wird 8 Stunden bei 80 °C geruehrt, darauf in Wasser gegossen, mit Essigester ausgeruehrt und filtriert. Als Rueckstand verbleiben 20.4 g 2-[3-(4-Benzamido-piperidino)-propoxy]-benzamidoxin-Hydrochlorid (68 % d. Th.) vom Schmp. 193-194 °C.

## Beispiel 12

2-[3-(4-Benzamido-piperidino)-propoxy]-benzamidin-Hydrochlorid

Eine Mischung aus 38.0 g (0.088 mol) 2-[3-(4-Benzamido-piperidino)-propoxy]-benzamidoxim-Hydrochlorid (Beispiel 11), 500 ml Ethanol und 20 ml Raney-Nickel wird 6 Stunden bei 70 °C und 30 bar Wasserstoffdruck hydriert. Nach dem Filtrieren engt man ein, chromatographiert an einer Kieselgelsaeule (Laufmittel : Methylenchlorid/Methanol) und engt die zweite Fraktion ein. Es verbleiben 16.2 g 2-[3-(4-Benzamido-piperidino)-propoxy]-benzamidin-Hydrochlorid (44 % d. Th.) vom Schmp. 212-215 °C.

## Beispiel 13

2-⟨2-[3-(4-Benzamido-piperidino)-propoxy]-phenyl⟩-4-hydroxy-pyrimidin-5-carbonsaeureethylester-Hydrochlorid

Eine Mischung aus 7.6 g (0.018 mol) 2-[3-(4-Benzamido-piperidino)-propoxy]-benzamidin-Hydrochlorid (Beispiel 12), 4.3 g Ethoxy-methylenmalonsaeurethylester und 100 ml Ethanol wird 2 Stunden zum Rueckfluss erhitzt, eingeengt und der Rueckstand aus Ethanol umkristallisiert. Man erhaelt 6.9 g 2-⟨2-[3-(4-Benzamido-piperidino)-propoxy]-phenyl⟩-4-hydroxy-pyrimidin-5-carbonsaeure-ethylester-Hydrochlorid (70 % d. Th.) vom Schmp. 250-252 °C.

Beispiel 14

2-⟨2-[3-(4-Benzamido-piperidino)-propoxy]-phenyl⟩-4-hydroxy-pyrimidin-5-carbonsaeure

In analoger Weise wie in Beispiel 7 beschrieben erhaelt man durch alkalische Verseifung von 2-⟨2-[3-(4-Benzamido-piperidino)-propoxy]-phenyl⟩-4-hydroxy-pyrimidin-5-carbonsaeureethylester-Hydrochlorid und anschliessendes Neutralisieren die Titelverbindung in 66 % Ausbeute vom Schmp. 145-146 °C (aus Ethanol).

Beispiel 15

| Wirkstoffhaltige Tabletten | | Für 1 Tabl. | Für 100.000 Tabl. |
|---|---|---|---|
| I | Wirkstoff 5-⟨2-[3-(4-Benzamido-piperidino)-propoxy]-phenyl⟩-1H-tetrazol | 10,000 mg | 1,000 kg |
| | Lactose | 67,000 mg | 6,700 kg |
| | Maisstärke | 35,000 mg | 3,500 kg |
| II | Polyvinylpyrrolidon, Mol.-Gew. 30.000 | 3,000 mg | 0,300 kg |
| III | Natriumcarboxymethylamylopektin | 4,000 mg | 0,400 kg |
| | Cellulosepulver | 20,000 lg | 2,000 kg |
| | Magnesiumstearat | 1,000 mg | 0,100 kg |
| | | 140,000 mg | 14,000 kg |
| | Wasser zum Granulieren | | 1,000 kg |

Herstellung : Die Substanzen I werdem mit der wäßrigen Lösung aus II granuliert, getrocknet und gesiebt. Das Granulat wird mit den Substanzen unter III zur Tablettiermasse gemischt. Die Tablettierung erfolgt zu Tabletten von 7 mm Durchmesser und 140 mg Gewicht.

Versuchsprotokoll

Methode

Die Versuchsanordnung zur Messung des Bronchospasmus entsprach im Prinzip der von KONZETT und RÖSSLER (1940) in der Modifikation nach COLLIER u.a. (1960). Tieren in mit 40 mg/kg Pentobarbital-Natrium i. p. herbeigeführter Narkose wurde eine Y-Kanüle in die Trachea und eine Venenkanüle in die V. jugularis eingebunden. Eine in die A. carotis eingebundene Kanüle führte zu einer Meßkammer (Statham, P 23 Db), mit welcher der arterielle Druck über eine Gleichspannungsmeßbrücke aufgezeichnet wurde. Mit einer Atempumpe wurde den Ratten ein Luftvolumen von, je nach Größe der Tiere, 1-2 ml 64 mal, den Meerschweinchen von 7-15 ml 72 mal pro Minute zugeführt. Die Narkose mußte so tief sein, daß eine spontane Gegenatmung nicht auftrat. Beim Auftreten eines Bronchospasmus, ausgelöst durch i. v.-Gabe des Antigens, konnte bei gleichbleibendem Beantmungsvolumen das gesamte angebotene Luftvolumen nicht mehr in die Lunge strömen und floß teilweise durch einen Seitenarm über ein Ventil ab. Die von der Firma BASILE entwickelte Methode weicht infofern von der ursprünglichen von KONZETT und RÖSSLER beschriebenen Methode ab, als dieses Luftvolumen indirekt über die Strömungsgeschwindigkeit mit Hilfe eines Thermoelementes gemessen wird. Die Stärke eines Bronchospasmus konnte dann als prozentuale Abnahme des verfügbaren Bronchialvolumens beschrieben werden. Den theoretisch maximal möglichen Bronchospasmus erhielt man durch Abklemmen des Beatmungsschlauches am Tier, so daß die gesamte zugeführte Luft durch das Ventil abströmte.

Alle 30 sec wurde durch ein Relais das Ventil für 8 sec geschlossen und das gesamte Volumen in die Lunge gepumpt, um die Bronchen und Alveolen wieder zu entfalten und so einen ausreichenden Gasaustausch zu ermöglichen. Die prozentuale Abnahme des verfügbaren Bronchialvolumens (X) wurde nach folgender Formel errechnet :

$$X = \frac{b - a}{m - a} \times 100$$

a) = Ausschlag des Schreibers bei Versuchsbeginn in mm.

b) = Höhe des Ausschlages zu einem bestimmten Zeitpunkt in mm.

m) = Maximaler Ausschlag in mm nach Abklemmen des Beatmungsschlauches am Tier.

COLLIER, H.O.J., J.A. HOLGATE, M. SCHACHTER : THE BRONCHOCONSTRICTOR ACTION OF BRADYKININ IN THE GUINEA-PIG., Brit. J. Pharmacol. *15*, 290 (1960).

KONZETT, H. und R. RÖSSLER :

Versuchsanordnung zu Untersuchungen an der Bronchialmuskulatur, Naunyn Schmiedebergs Arch. exp. Path. Pharmak. *195*, 71-74 (1940).

Präparation des Antiserums

Das Antigen war 2 × kristallisiertes Ovalbumin. Gleiche Mengen einer Lösung des Antigens in physiologischer Kochsalzlösung und komplettem Freund'schen Adjuvans wurden emulgiert und 2 × 0,15 ml i. m. bei männlichen Meerschweinchen injiziert. Die Tiere wurden entblutet und das gepoolte Serum bei − 20 °C aufbewahrt. Zur passiven Sensibilisierung wurden Meerschweinchen 0,5 ml des Antiserum 1 : 50 verdünnt 24-48 Stunden vor Auslösen des Bronchospasmus i. v. injiziert.

(DAVIES und JOHNSON :

Int. Arch. Allergy 41, 648-654 (1971))

Die erfindungsgemäßen Verbindungen wurden 5 Minuten vor Zugabe des Antigens i. v. appliziert.

Die Inhibierung der Bronchospasmen (Br-Sp) in % wurden 3 Minuten nach der Antigenzugabe im Vergleich zur Kontrollgruppe bestimmt.

Inhibierung des Antigen-induzierten Bronchosparums (Br-Sp) in passiv sensibilisierten Meerschweinchen

| Beispiel Nr. | % Inhibierung Br-Sp | |
|---|---|---|
| | Dosis mg/kg i.v. | % |
| 7 | 1.5 | 45 |
| 9 | 0.1 | 56 |
| 2 a | 3.0 | 71 |
| 2 b | 0.38 | 67 |
| 10 g | 0.75 | 71 |
| Aminophyllin* | 24 | 29 |

* Aminophyllin = 2 Mol Theophyllin + 1 Mol Ethylendiamin.

**Ansprüche**

1. N-Phenoxyalkylpiperidin-Derivate der allgemeinen Formel I

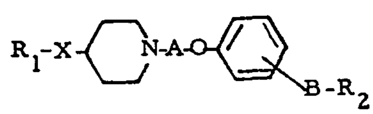

(I)

in der

$R_1$ Wasserstoff, einen $C_1$-$C_7$ Alkanoyl-Rest, einen Trifluoracetyl-Rest, einen Phenacetyl-Rest, einen $C_3$-$C_7$ Cycloalkylcarbonyl-Rest, einen Furancarbonyl-, Thiophencarbonyl- oder Pyridincarbonyl-Rest, einen Phenyl- oder Naphthyl-Rest, oder einen Benzoyl-Rest, der durch Halogen, Hydroxyl, $C_1$-$C_6$ Alkyl, $C_1$-$C_6$ Alkoxy, $C_1$-$C_6$ Alkoxycarbonyl, Acetyloxy, Carboxyl, Nitro, Carbamoyl, Amino, Nitril, Trifluormethyl, Methylmercapto, Methylsulfinyl, Methylsulfonyl, Acetyl, Benzoyl, Acetylamino, Hydroxymethyl oder $C_1$-$C_6$ Alkoxy $C_1$-$C_6$ Alkyl substituiert sein kann,

$R_2$ eine Hydroxymethylgruppe, eine Cyanogruppe, eine Amidinogruppe, die gewünschtenfalls durch Hydroxyl substituiert sein kann, eine 1H-Tetrazol-5-yl-gruppe oder eine Gruppe —CO—$R_3$,

$R_3$ eine Hydroxygruppe, eine $C_1$-$C_6$ Alkoxygruppe oder eine Aminogruppe, die gewünschtenfalls durch 1H-Tetrazol-5-yl substituiert sein kann,

X eine Iminogruppe oder eine Oxymethylengruppe,

A einen Trimethylen-Rest und

B einen Valenzstrich oder eine 4-Hydroxy-pyrimidin-2,5-diyl-gruppe bedeuten, sowie deren pharmakologisch verträgliche Salze.

2. Verbindungen der allgemeinen Formel I gemäß Anspruch 1, bei denen $R_1$, $R_2$, $R_3$, A und B die angegebenen Bedeutungen haben und X eine Iminogruppe darstellt, sowie deren pharmakologisch verträgliche Salze.

3. Verbindungen der allgemeinen Formel I gemäß Anspruch 2, bei denen $R_1$, $R_2$, $R_3$, A und X die angegebenen Bedeutungen haben und B einen Valenzstrich bedeutet, sowie deren pharmakologisch verträgliche Salze.

4. Verbindungen der allgemeinen Formel I gemäß Anspruch 2, bei denen $R_1$, $R_2$, $R_3$, A und X die angegebenen Bedeutungen haben und B eine 4-Hydroxypyrimidin-2,5-diyl-gruppe bedeutet, sowie deren pharmakologisch verträgliche Salze.

5. Verbindungen der allgemeinen Formel I gemäß Anspruch 1, bei denen $R_1$, $R_3$, X und A die angegebenen Bedeutungen haben, B einen Valenzstrich und $R_2$ eine 1H-Tetrazol-5-yl-gruppe darstellen, sowie deren pharmakologisch verträgliche Salze.

6. Verfahren zur Herstellung von N-Phenoxyalkylpiperidin-Derivaten der allgemeinen Formel I

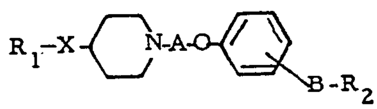

(I)

in der

$R_1$ Wasserstoff, einen $C_1$-$C_7$ Alkanoyl-Rest, einen Trifluoracetyl-Rest, einen Phenacetyl-Rest, einen $C_3$-$C_7$ Cycloalkylcarbonyl-Rest, einen Furancarbonyl-, Thiophencarbonyl- oder Pyridincarbonyl-Rest, einen Phenyl- oder Naphthyl-Rest, oder einen Benzoyl-Rest, der durch Halogen, Hydroxyl, $C_1$-$C_6$ Alkyl, $C_1$-$C_6$ Alkoxy, $C_1$-$C_6$ Alkoxycarbonyl, Acetyloxy, Carboxyl, Nitro, Carbamoyl, Amino, Nitril, Trifluormethyl, Methylmercapto, Methylsulfinyl, Methylsulfonyl, Acetyl, Benzoyl, Acetylamino, Hydroxymethyl oder $C_1$-$C_6$ Alkoxy $C_1$-$C_6$ alkyl substituiert sein kann,

$R_2$ eine Hydroxymethylgruppe, eine Cyanogruppe, eine Amidinogruppe, die gewünschtenfalls durch Hydroxyl substituiert sein kann, eine 1H-Tetrazol-5-yl-gruppe oder eine Gruppe —CO—$R_3$,

$R_3$ eine Hydroxygruppe, eine $C_1$-$C_6$ Alkoxygruppe oder eine Aminogruppe, die gewünschtenfalls durch 1H-Tetrazol-5-yl substituiert sein kann,

X eine Iminogruppe oder eine Oxymethylengruppe,

A einen Trimethylen-Rest und

B einen Valenzstrich oder eine 4-Hydroxy-pyrimidin-2,5-diyl-gruppe bedeuten, sowie deren pharmakologisch verträglichen Salzen, dadurch gekennzeichnet, daß man in an sich bekannter Weise eine Verbindung der allgemeinen Formel II

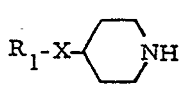

(II)

in der

$R_1$ und X die obengenannte Bedeutung haben, mit einer Verbindung der allgemeinen Formel III

$$Y_1—A—Y_2 \qquad (III)$$

in der

$Y_1$ und $Y_2$ reaktive Reste bedeuten und A die obengenannte Bedeutung hat, und einer Verbindung der allgemeinen Formel IV

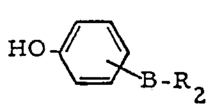

(IV)

in der

B und $R_2$ die obengenannte Bedeutung haben, umsetzt, anschließend gewünschtenfalls die Gruppe $R_1$ oder die Gruppe —B—$R_2$ durch an sich bekannte Verfahren in eine andere, der obengenannten Bedeutung entsprechende Gruppe $R_1$ oder —B—$R_2$ umwandelt und das so erhaltene Reaktionsprodukt gewünschtenfalls in ein pharmakologisch verträgliches Salz überführt.

7. Verbindungen der allgemeinen Formel I und deren pharmakologisch verträgliche Salze zur Verwendung als Arzneimittel mit antiallergischer, antiödematöser und antiphlogistischer Wirkung.

8. Arzneimittel, gekennzeichnet durch einen Gehalt an Verbindungen der allgemeinen Formel I bzw. deren pharmakologisch verträglichen Salzen.

**Claims**

1. N-Phenoxyalkylpiperidine derivatives of the general formula I

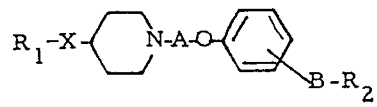

(I)

in which

$R_1$ signifies hydrogen, a $C_1$-$C_7$ alkanoyl radical, a trifluoroacetyl radical, a phenacetyl radical, a $C_3$-$C_7$ cycloalkylcarbonyl radical, a furanecarbonyl, thiophenecarbonyl or pyridinecarbonyl radical, a phenyl or naphthyl radical, or a benzoyl radical, which can be substituted by halogen, hydroxyl, $C_1$-$C_6$ alkyl, $C_1$-$C_6$ alkoxy, $C_1$-$C_6$ alkoxycarbonyl, acetyloxy, carboxyl, nitro, carbamoyl, amino, nitrile, trifluoromethyl, methylthio, methylsulphinyl, methylsulphonyl, acetyl, benzoyl, acetylamino, hydroxymethyl or $C_1$-$C_6$ alkoxy $C_1$-$C_6$ alkyl,

$R_2$ signifies a hydroxymethyl group, a cyano group, an amidino group, which, if desired, can be substituted by hydroxyl, a 1H-tetrazol-5-yl group or a group —CO—$R_3$,

$R_3$ signifies a hydroxyl group, a $C_1$-$C_6$ alkoxy group or an amino group, which, if desired, can be substituted by 1H-tetrazol-5-yl,

X signifies an imino group or an oxymethylene group,

A signifies a trimethylene radical and

B signifies a valency bond or a 4-hydroxypyrimidin-2,5-diyl group ; as well as their pharmacologically acceptable salts.

2. Compounds of the general formula I according to claim 1, in which $R_1$, $R_2$, $R_3$, A and B have the given meanings and X represents an imino group ; as well as their pharmacologically acceptable salts.

3. Compounds of the general formula I according to claim 2, in which $R_1$, $R_2$, $R_3$, A and X have the given meanings and B signifies a valency bond ; as well as their pharmacologically acceptable salts.

4. Compounds of the general formula I according to claim 2, in which $R_1$, $R_2$, $R_3$, A and X have the given meanings and B signifies a 4-hydroxypyrimidine-2,5-diyl group ; as well as their pharmacologically acceptable salts.

5. Compounds of the general formula I according to claim 1, in which $R_1$, $R_3$, X and A have the given meanings, B represents a valency bond and $R_2$ a 1H-tetrazol-5-yl group ; as well as their pharmacologically acceptable salts.

6. Process for the preparation of N-phenoxyalkylpiperidine derivatives of the general formula I

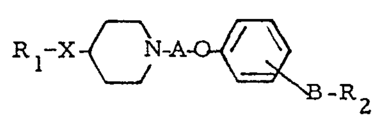

(I)

in which

$R_1$ signifies hydrogen, a $C_1$-$C_7$ alkanoyl radical, a trifluoroacetyl radical, a phenacetyl radical, a $C_3$-$C_7$ cycloalkylcarbonyl radical, a furanecarbonyl, thiophenecarbonyl or pyridinecarbonyl radical, a phenyl or naphthyl radical, or a benzoyl radical, which can be substituted by halogen, hydroxyl, $C_1$-$C_6$ alkyl, $C_1$-$C_6$ alkoxy, $C_1$-$C_6$ alkoxycarbonyl, acetoloxy, carboxyl, nitro, carbamoyl, amino-, nitrile, trifluoromethyl, methylthio, methylsulphinyl, methylsulphonyl, acetyl, benzoyl, acetylamino, hydroxymethyl or $C_1$-$C_6$ alkoxy $C_1$-$C_6$ alkyl,

$R_2$ signifies a hydroxymethyl group, a cyano group, an amidino group, which, if desired, can be substituted by hydroxyl, a 1H-tetrazol-5-yl group or a group —CO—$R_3$,

$R_3$ signifies a hydroxyl group, a $C_1$-$C_6$ alkoxy group or an amino group, which, if desired, can be substituted by 1H-tetrazol-5-yl,

X signifies an imino group or an oxymethylene group,

A signifies a trimethylene radical and

B signifies a valency bond or a 4-hydroxypyrimidin-2,5-diyl group ; as well as of their pharmacologically acceptable salts, characterised in that, in per se known manner, one reacts a compound of the general formula II

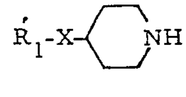

(II)

in which

$R_1$ and X have the above-mentioned meaning, with a compound of the general formula III

$Y_1—A—Y_2$ (III)

in which

$Y_1$ and $Y_2$ signify reactive residues and A has the above-mentioned meaning, and a compound of the general formula IV

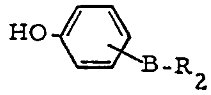

(IV)

in which

B and $R_2$ have the above-mentioned meaning, subsequently, if desired, the group $R_1$ or the group $—B—R_2$ is converted by per se known processes into another group $R_1$ or $—B—R_2$ according to the above-mentioned meaning and the so obtained reaction product is, if desired, converted into a pharmacologically acceptable salt.

7. Compounds of the general formula I and their pharmacologically acceptable salts for use as medicaments with anti-allergic, anti-oedematous and antiphlogistic action.

8. Medicaments, characterised by a content of compounds of the general formula I or of the pharmacologically acceptable salts.

**Revendications**

1. Dérivés de la N-phénoxyalkylpipéridine de formule générale I :

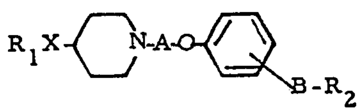

(I)

dans laquelle

$R_1$ est de l'hydrogène, un reste alcanoyle $C_1$-$C_7$, un reste trifluoro-acétyle, un reste phénacétyle, un reste cyclo-alkylcarbonyle $C_3$-$C_7$, un reste furanne-carbonyle, thiophène-carbonyle ou pyridine-carbonyle, un reste phényle ou naphtyle ou un reste benzoyle pouvant être substitué par de l'halogène, de l'hydroxyle, de l'alkyle $C_1$-$C_6$, de l'alcoxy $C_1$-$C_6$, de l'alcoxycarbonyle $C_1$-$C_6$, de l'acétyloxy, du carboxyle, du nitro, du carbamoyle, de l'amino, du nitrile, du trifluorométhyle, du méthylmercapto, du méthylsulfinyle, du méthylsulfonyle, de l'acétyle, du benzoyle, de l'acétylamino, de l'hydroxyméthyle ou de l'alcoxy $C_1$-$C_6$ alkyle $C_1$-$C_6$,

$R_2$ est un groupe hydroxyméthyle, un groupe cyano, un groupe amidino, éventuellement substitués par de l'hydroxyle, un groupe 1H-tétrazole-5-yle ou un groupe $—CO—R_3$,

$R_3$ est un groupe hydroxyle, un groupe alcoxy $C_1$-$C_6$ ou un groupe amino, éventuellement substitué par 1H-tétrazole-5-yle,

X est un groupe imino ou un groupe oxyméthylène,

A est un reste triméthylène et

B est un trait de valence ou un groupe 4-hydroxy-pyrimidine-2,5-di-yle, ainsi que leurs sels pharmacologiquement tolérables.

2. Dérivés de formule générale I selon la revendication 1, dans lesquels $R_1$, $R_2$, $R_3$ A et B ont les significations indiquées et X est un groupe imino, ainsi que leurs sels pharmacologiquement tolérables.

3. Dérivés de formule générale I selon la revendication 2, dans lesquels $R_1$, $R_2$, $R_3$, A et X ont les significations indiquées et B est un trait de valence, ainsi que leurs sels pharmacologiquement tolérables.

4. Dérivés de formule générale I selon la revendication 2, dans lesquels $R_1$, $R_2$, $R_3$, A et X ont les significations indiquées et B est un groupe 4-hydroxy-pyrimidine-2,5-di-yle, ainsi que leurs sels pharmacologiquement tolérables.

5. Dérivés de formule générale I selon la revendication 1, dans lesquels $R_1$, $R_3$, X et A ont les significations indiquées, B est un trait de valence et $R_2$ un groupe 1H-tétrazole-5-yle, ainsi que leurs sels pharmacologiquement tolérables.

6. Procédé de préparation de dérivés de la N-phénoxyalkylpipéridine de formule générale I :

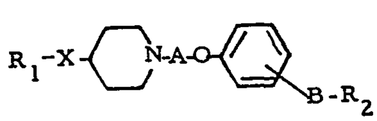

(I)

dans laquelle

$R_1$ est de l'hydrogène, un reste alcanoyle $C_1$-$C_7$, un reste trifluoro-acétyle, un reste phénacétyle, un

reste cyclo-alkylcarbonyle $C_3$-$C_7$, un reste furanne-carbonyle, thiophène-carbonyle ou pyridine-carbonyle, un reste phényle ou naphtyle ou un reste benzoyle pouvant être substitué par de l'halogène, de l'hydroxyle, de l'alkyle $C_1$-$C_6$, de l'alcoxy $C_1$-$C_6$, de l'alcoxycarbonyle $C_1$-$C_6$, de l'acétyloxy, du carboxyle, du nitro, du carbamoyle, de l'amino, du nitrile, du trifluorométhyle, du méthylmercapto, du méthylsulfinyle, du méthylsulfonyle, de l'acétyle, du benzoyle, de l'acétylamino, de l'hydroxyméthyle ou de l'alcoxy $C_1$-$C_6$ alkyle $C_1$-$C_6$.

$R_2$ est un groupe hydroxyméthyle, un groupe cyano, un groupe amidino, éventuellement substitués par de l'hydroxyle, un groupe 1H-tétrazole-5-yle ou un groupe —CO—$R_3$,

$R_3$ est un groupe hydroxyle, un groupe alcoxy $C_1$-$C_6$ ou un groupe amino, éventuellement substitué par 1H-tétrazole-5-yle,

X est un groupe imino ou un groupe oxyméthylène

A est un reste triméthylène et

B est un trait de valence ou un groupe 4-hydroxy-pyrimidine-2,5-di-yle, ainsi que leurs sels pharmacologiquement tolérables caractérisé en ce que de façon en soi connue on fait réagir un composé de formule générale II

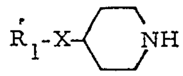

(II)

dans laquelle

$R_1$ et X ont la signification ci-dessus indiquée, avec un composé de formule générale III

$$Y_1—A—Y_2 \quad (III)$$

dans laquelle

$Y_1$ et $Y_2$ sont des restes réactifs et A a la signification ci-dessus indiquée, et avec un composé de formule générale IV

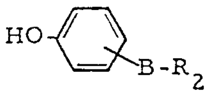

(IV)

dans laquelle

B et $R_2$ ont la signification ci-dessus indiquée, et ensuite, on transforme éventuellement au moyen de procédés en soi connus le groupe $R_1$ ou le groupe —B—$R_2$ en un autre groupe $R_1$ ou —B—$R_2$ correspondant à la définition ci-dessus, et on transforme le produit de réaction ainsi obtenu éventuellement en un sel pharmacologiquement acceptable.

7. Composés de formule générale I et leurs sels pharmacologiquement tolérables pour l'utilisation comme médicament ayant des activités anti-allergique, anti-œdémateuse et anti-phlogistique.

8. Médicament caractérisé par une teneur en composés de formule générale I ou leurs sels pharmacologiquement tolérables.